Europäisches Patentamt
European Patent Office
Office européen des brevets

(11) Veröffentlichungsnummer: **0 282 692 A1**

# (12) EUROPÄISCHE PATENTANMELDUNG

(21) Anmeldenummer: **88100651.4**

(51) Int. Cl.⁴: **C07D 249/08**, A01N 43/653

(22) Anmeldetag: **19.01.88**

(30) Priorität: **31.01.87 DE 3702920**

(43) Veröffentlichungstag der Anmeldung: **21.09.88 Patentblatt 88/38**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kranz, Eckart, Dr.**
**Am Acker 9**
**D-5600 Wuppertal 1(DE)**
Erfinder: **Jautelat, Manfred, Dr.**
**Müllerbaum 28**
**D-5093 Burscheid(DE)**
Erfinder: **Brandes, Wilhelm, Dr.**
**Eichendorffstrasse 3**
**D-5653 Leichlingen 1(DE)**
Erfinder: **Hänssler, Gerd, Dr.**
**Am Arenzberg 58a**
**D-5090 Leverkusen 3(DE)**
Erfinder: **Reinecke, Paul, Dr.**
**Steinstrasse 8**
**D-5090 Leverkusen 1(DE)**

(54) **Substituierte Hydroxy-azolyl-ketone.**

(57) Neue substituierte Hydroxy-azolyl-ketone der Formel

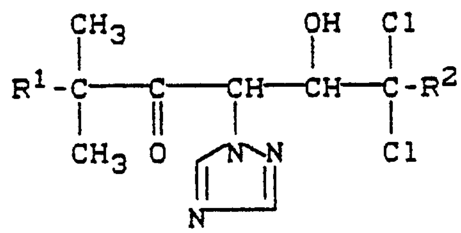

(I)

in welcher
$R^1$ für geradkettiges oder verzweigtes Alkyl mit 2 bis 5 Kohlenstoffatomen oder Alkenyl mit 2 bis 5 Kohlenstoffatomen steht und
$R^2$ für Wasserstoff, Halogen oder gegebenenfalls einfach oder mehrfach durch Halogen substituiertes geradkettiges oder verzweigtes Alkyl mit 1 bis 5 Kohlenstoffatomen steht,
sowie deren Säureadditions-Salze und Metallsalz-Komplexe,
ein Verfahren zur Herstellung der neuen Stoffe und deren Verwendung als Fungizide.

EP 0 282 692 A1

## Substituierte Hydroxy-azolyl-ketone

Die vorliegende Erfindung betrifft neue substituierte Hydroxy-azolyl-ketone, ein Verfahren zu ihrer Herstellung und ihre Verwendung als Fungizide.

Es ist bereits bekannt geworden, daß substituierte Phenoxytriazolyl-keto-bzw. -hydroxy-Derivate fungizide Eigenschaften aufweisen (vergleich EP-OS 0 007 505). So können z.B. 1,1-Dichlor-2-hydroxy-3-(1,2,4-triazol-1-yl)-5,5-dimethyl-hexan-4-on und 2,3,3-Trichlor-4-hydroxy-5-(1,2,4-triazol-1-yl)-7,7-dimethyl-octan-6-on zur Bekämpfung von Pilzen eingesetzt werden. Die Wirksamkeit dieser Stoffe ist jedoch, vor allem bei niedrigen Aufwandmengen und Konzentrationen, nicht immer ganz befriedigend.

Es wurden nun neue substituierte Hydroxy-azolyl-ketone der Formel (I)

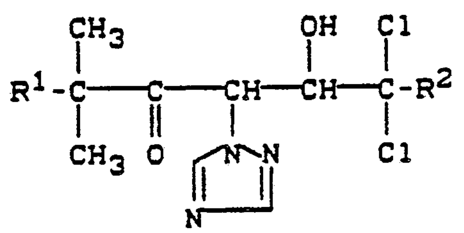

(I)

in welcher

$R^1$ für geradkettiges oder verzweigtes Alkyl mit 2 bis 5 Kohlenstoffatomen oder Alkenyl mit 2 bis 5 Kohlenstoffatomen steht und

$R^2$ für Wasserstoff, Halogen oder gegebenenfalls einfach oder mehrfach durch Halogen substituiertes geradkettiges oder verzweigtes Alkyl mit 1 bis 5 Kohlenstoffatomen steht,

sowie deren Säureadditions-Salze und Metallsalz-Komplexe gefunden.

Die neuen substituierten Hydroxy-azolyl-ketone der Formel (I) besitzen zwei asymmetrisch substituierte Kohlenstoffatome; sie können deshalb in der erythro-wie in der threo-Form vorliegen. In beiden Fällen liegen sie vorwiegend als Racemate vor. Die Erfindung betrifft sowohl die Isomerengemische als auch die einzelnen Isomeren.

Weiterhin wurde gefunden, daß man substituierte Hydroxyazolyl-ketone der Formel (I) bzw. deren Säureadditions-Salze und Metallsalz-Komplexe erhält, wenn man α-Azolylketone der Formel (II)

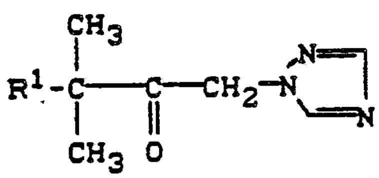

(II)

in welcher

$R^1$ die oben angegebene Bedeutung hat,

mit Aldehyden der Formel (III)

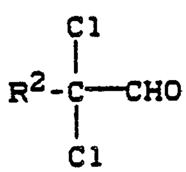

(III)

in welcher

$R^2$ die oben angegebene Bedeutung hat,

in Gegenwart eines Verdünnungsmittels und gegebenenfalls in Gegenwart eines Katalysators umsetzt und gegebenenfalls anschließend an die so erhaltenen Verbindungen der Formel (I) eine Säure oder ein Metallsalz addiert. In manchen Fällen können die Aldehyde der Formel (III) auch in Form ihrer Hydrate oder

Halbacetale eingesetzt werden.

Außerdem wurde gefunden, daß sich die neuen substituierten Hydroxy-azolyl-ketone der Formel (I) sowie deren Säureadditions-Salze und Metallsalz-Komplexe durch sehr gute fungizide Eigenschaften auszeichnen.

Überraschenderweise besitzen die erfindungsgemäßen substituierten Hydroxy-azolyl-ketone der Formel (I) sowie deren Säureadditions-Salze und Metallsalz-Komplexe eine bessere fungizide Wirksamkeit als das 1,1-Dichlor-2-hydroxy-3-(1,2,4-triazol-1-yl)-5,5-dimethyl-hexan-4-on und das 2,3,3-Trichlor-4-hydroxy-5-(1,2,4-triazol-1-yl)-7,7-dimethyl-octan-6-on, welches konstitutionell ähnliche, vorbekannte Wirkstoffe gleicher Wirkungsrichtung sind.

Die erfindungsgemäßen substituierten Hydroxy-azolyl-ketone sind durch die Formel (I) allgemein definiert. Bevorzugt sind diejenigen Verbindungen der Formel (I), in denen

$R^1$ für geradkettiges oder verzweigtes Alkyl mit 2 bis 4 Kohlenstoffatomen oder Alkenyl mit 2 bis 4 Kohlenstoffatomen steht und

$R^2$ für Wasserstoff, Fluor oder Chlor, gegebenenfalls einfach oder mehrfach durch Fluor und/oder Chlor substituiertes geradkettiges oder verzweigtes Alkyl mit 1 bis 4 Kohlenstoffatomen steht.

Besonders bevorzugt sind diejenigen substituierten Hydroxy-azolyl-ketone der Formel (I), in denen

$R^1$ für geradkettiges oder verzweigtes Alkyl mit 2 oder 3 Kohlenstoffatomen, Vinyl oder Allyl steht und

$R^2$ für Wasserstoff, Chlor, gegebenenfalls einfach oder mehrfach durch Chlor substituiertes geradkettiges oder verzweigtes Alkyl mit 1 bis 3 Kohlenstoffatomen steht.

Bevorzugte erfindungsgemäße Verbindungen sind auch Additionsprodukte aus Säuren und denjenigen substituierten Hydroxy-azolyl-ketonen der Formel (I), in denen $R^1$ und $R^2$ diejenigen Bedeutungen haben, die im Zusammenhang mit der Beschreibung der erfindungsgemäßen Stoffe bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt-und der I. und II. sowie IV. bis VIII. Nebengruppe des Periodensystems der Elemente und denjenigen substituierten Hydroxy-azolyl-ketonen der Formel (I), in denen $R^1$ und $R^2$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenphysiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und schwefelsäure.

Verwendet man beispielsweise 1-(1,2,4-Triazol-1-yl)-3,3,4-trimethyl-pentan-2-on und Chloral als Ausgangssubstanzen, so kann der Verlauf des erfindungsgemäßen Verfahrens durch das folgende Formelschema wiedergegeben werden:

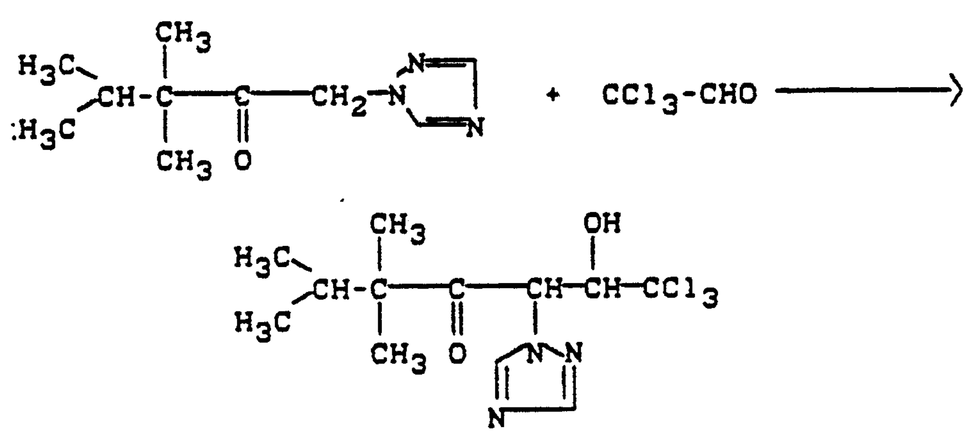

Die für das erfindungsgemäße Verfahren als Ausgangsstoffe zu verwendenden α-Azolyl-ketone sind durch die Formel (II) allgemein definiert. In dieser Formel hat $R^1$ bevorzugt bzw. besonders bevorzugt

diejenigen Bedeutungen, die bereits im Zusammenhang mit der Beschreibung der erfindungsgemäßen Stoffe der Formel (I) als bevorzugt bzw. besonders bevorzugt für diesen Rest genannt wurden.

Die α-Azolyl-ketone der Formel (II) sind teilweise bekannt (vergleiche EP-OS 0 007 505). Man erhält die Verbindungen der Formel (II), indem man

a) Halogenketone der Formel (IV)

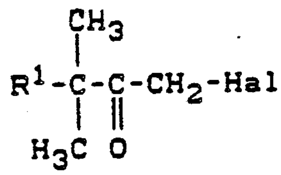

(IV)

in welcher
$R^1$ die oben angegebene Bedeutung hat und
Hal für Chlor oder Brom steht,
mit 1,2,4-Triazol in Gegenwart eines inerten organischen Lösungsmittels, wie beispielsweise Aceton, und in Gegenwart eines Säurebindemittels, wie beispielsweise Natriumcarbonat bei Temperaturen zwischen 20 und 100°C umsetzt,
oder

b) Hydroxyketone der Formel (V)

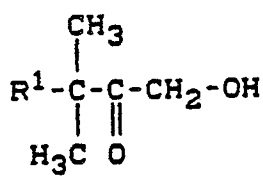

(V)

in welcher
$R^1$ die oben angegebene Bedeutung hat,
mit Thionyl-bis-azol der Formel (VI)

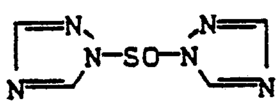

(VI)

in Gegenwart eines polaren Lösungsmittels, wie beispielsweise Acetonitril bei Temperaturen zwischen 20 und 150°C umsetzt.

Die Verbindungen der Formeln (IV) bis (VI) sind bekannt oder lassen sich nach bekannten Verfahren in einfacher Weise herstellen.

Die außerdem für das erfindungsgemäße Verfahren als Ausgangsstoffe zu verwendenden Aldehyde sind durch die Formel (III) allgemein definiert. In dieser Formel hat $R^2$ bevorzugt bzw. besonders bevorzugt diejenigen Bedeutungen die bereits im Zusammenhang mit der Beschreibung der erfindungsgemäßen Stoffe der Formel (I) als bevorzugt bzw. besonders bevorzugt für diesen Rest angegeben wurden.

Die Aldehyde der Formel (III), deren Hydrate bzw. Halbacetale sind allgemein bekannte Verbindungen der organischen Chemie. Als Beispiele seien genannt:
Chloral, Dichlorfluoracetaldehyd, 2,2,3-Trichlorbutyraldehyd, 2,2-Cichlorpropionaldehyd, Glyoxylsäuremethylester, Glyoxylsäureethylester, dichloracetaldehyd, Chloracetaldehyd.

Als Lösungsmittel kommen für die Durchführung des erfindungsgemäßen Verfahrens vorzugsweise inerte organische Lösungsmittel in Frage, Hierzu gehören vorzugsweise Alkohole, wie Methanol und Ethanol, sowie deren Mischungen mit Wasser; Ether, wie Tetrahydrofuran und Dioxan; Nitrile, wie Acetonitril und Propionitril; halogenierte aliphatische und aromatische Kohlenwasserstoffe, wie Methylenchlorid, Tetrachlorkohlenstoff, Chloroform, Chlorbenzol und Dichlorbenzol; sowie Eisessig.

Das erfindungsgemäße Verfahren wird gegebenenfalls in Gegenwart eines Katalysators durchgeführt. Man kann alle üblicherweise verwendbaren sauren und insbesondere basischen Katalysatoren einsetzen. Hierzu gehören vorzugsweise Lewis-Säuren, wie z.B. Eisen-(III)-chlorid, Eisen-(III)-bromid, Bortrifluorid, Bortrichlorid, Zinntetrachlorid oder Titantetrachlorid; Alkali-und Erdalkalihydroxide, wie Kaliumhydroxid,

Natriumhydroxid, Calciumhydroxid oder Bariumhydroxid; Alkalisalze, wie Kaliumcarbonat, Natriumcarbonat; Kaliumcyanid; sekundäres Natriumphosphat, Natriumacetat oder Natriumsulfit; sowie Alkoholate, wie Natrium-oder Kaliummethylat.

Die Reaktionstemperaturen können bei der Durchführung des erfindungsgemäßen Verfahrens in einem größeren Bereich variiert werden. Im allgemeinen arbeitet man zwischen 0 und 100°C, vorzugsweise bei Raumtemperatur bzw. dem Siedepunkt des jeweiligen Lösungsmittels.

Bei der Durchführung des erfindungsgemäßen Verfahrens arbeitet man vorzugsweise mit äquimolaren Mengen an Ausgangssubstanzen, wobei katalytische bis äquimolare Mengen an Katalysator eingesetzt werden. Die Isolierung der Verbindungen der Formel (I) erfolgt in üblicher Weise (vergleiche die Herstellungsbeispiele).

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen den Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metall salzes in Alkohol, z.B. Ethanol, und Hinzufügen zu Verbindungen der allgemeinen Formel (I). Man kann die Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel eingesetzt. Sie lassen sich vorzugsweise als Fungizide oder Mikrobizide verwenden.

Fungizide Mittel im Pflanzenschtz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botyrtis cinerea;
Septoria-Arten, wie beispielsweise Septoria nodorum;
Leptosphaeria-Arten, wie beispielsweise Leptosphaeria nodorum;

Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Kon zentrationen erlaubt eine Behandlung von oberirdischen Pflanzenteilen, von Pflanz-und Saatgut, und des Bodens.

Die erfindungsgemäßen Wirkstoffe können mit besonders gutem Erfolg protektiv gegen Sphaerotheca bei Gurken und Erysiphe bei Weizen eingesetzt werden.

Hervorzuheben ist, daß die erfindungsgemäßen Stoffe nicht nur protektiv, sondern auch systemisch eingesetzt werden können zur Bekämpfung von Pyricularia an Reis, gegen Getreidemehltau sowie gegen Cochliobolus sativus und Pyrenophora teres an Getreide.

Die Wirkstoffe können in Abhängigkeit von ihren jeweiligen physikalischen und/oder chemischen Eigenschaften in übliche Formulierungen überfeführt werden, wie Lösungen, Emulsionen, suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonderden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und/oder Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmenge an erfindungsgemäßen Wirkstoffen kann je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den folgenden Beispielen hervor.

Herstellungsbeispiele:

Beispiel 1

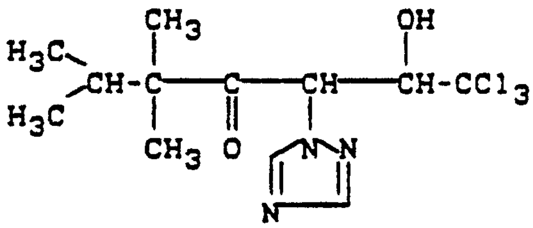

Zu einer Lösung aus 19,5 g (0,1 Mol) 1-(1,2,4-Triazol-1-yl)-3,3,4-trimethyl-pentan-2-on in 200 ml Tetrahydrofuran und 27,6 g gemahlenem Kaliumcarbonat werden 18,6 g (0,13 Mol) Chloral gegeben. Das Reaktionsgemisch wird 15 Stunden bei Raumtemperatur gerührt. Danach wird der Ansatz in Wasser gegossen, und es wird eine Stunde nachgerührt. Danach wird der anfallende Feststoff abgesaugt und aus Toluol umkristallisiert.

Man erhält 24,1 g (70,3 % der Theorie) 1,1,1-Trichlor-2-hydroxy-3-(1,2,4-triazol-1-yl)-5,5,6-trimethyl-heptan-4-on vom Schmelzpunkt 161-169°C.

Herstellung des Ausgangsstoffes

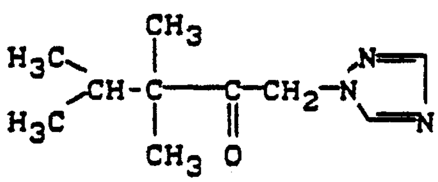

138 g (2,0 Mol) 1,2,4-Triazol werden bei Raumtemperatur portionsweise zu 303,6 g gemahlenem Kaliumcarbonat und 325 g (2,0 Mol) 1-Chlor-3,3,4-trimethyl-pentan-2-on in 500 ml Aceton gegeben. Der Ansatz wird 5,5 Stunden unter Rück fluß gerührt und dann auf Raumtemperatur abgekühlt. Das Reaktionsgemisch wird filtriert, mit Aceton nachgewaschen, und das Filtrat wird durch Abdestillieren des Lösungsmittels im Vakuum eingeengt. Der ölige Rückstand kristallisiert nach Zugabe von Ether.

Man erhält 337,7 g (74,4 % der Theorie) 3,3,4-Trimethyl-1-(1,2,4-triazol-1-yl)-pentan-2-on vom Schmelzpunkt 155-156°C.

In analoger Weise zu der im Beispiel 1 beschriebenen Methode und unter Berücksichtigung der Angaben in der Beschreibung zu dem erfindungsgemäßen Verfahren werden die nachfolgend aufgeführten Verbindungen der Formel (I)

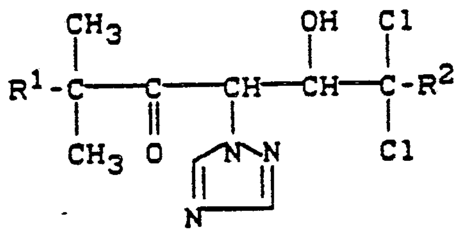

(I)

erhalten:

Tabelle I

| Bsp. Nr. | $R^1$ | $R^2$ | Schmelzpunkt [°C] |
|---|---|---|---|
| 2 | $-CH{=}CH_2$ | $-CH_3$ | 142-144 |
| 3 | $-CH{=}CH_2$ | $-CH_2-CH_2-CH_3$ | 131-132 |
| 4 | $-CH{=}CH_2$ | H | 168-170 |

Tabelle I(Fortsetzung)

| Bsp. Nr. | $R^1$ | $R^2$ | Schmelzpunkt [°C] |
|---|---|---|---|
| 5 | $-CH{=}CH_2$ | $-\underset{\substack{\vert\\Cl}}{CH}-CH_3$ | 205-207 |
| 6 | $-CH{=}CH_2$ | $-CH_2-CH_3$ | 138-140 |
| 7. | $i-C_3H_7$ | $-CH_2Cl$ | 148-150 |
| 8 | $i-C_3H_7$ | $-CH_2-CH_3$ | 124-126 |
| 9 | $i-C_3H_7$ | $-\underset{\substack{\vert\\Cl}}{CH}-CH_3$ | 210-212 |
| 10 | $i-C_3H_7$ | $-CH_2-CH_2-CH_3$ | 116-118 |

Verwendungsbeispiele"

In den folgenden Verwendungsbeispielen werden die nachstehend angegebenen Substanzen als Vergleichsverbindungen eingesetzt

(A) 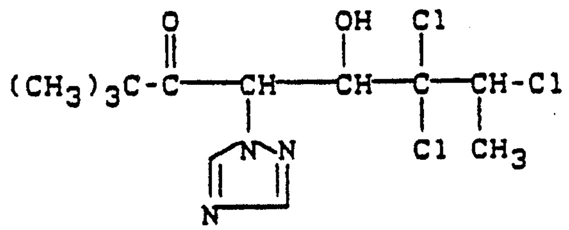

(B) 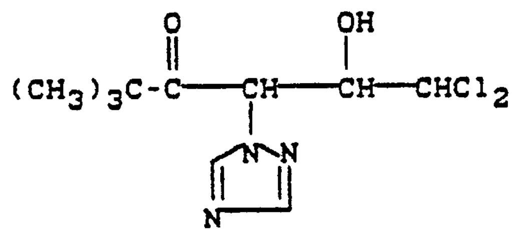

(Bekannt aus EP-OS 0 007 505)

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritabelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolge die Auswertung.

In diesem Test zeigen die erfindungsgemäßen Stoffe (1), (2), (3), (6), (8) und (10) eine bessere Wirksamkeit als die Vergleichssubstanzen (A) und (B).

Beispiel B

Sphaerotheca-Test (Gurke) / protektiv

Lösungsmittel: 4,7 Gewichtsteile Aceton
Emulgator: 0,3 Gewichtsteile Alkyl-arylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis

zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

In diesem Test zeigen die erfindungsgemäßen Stoffe (1), (6), (7), (8) und (10) eine bessere Wirksamkeit als die Vergleichssubstanzen (A) und (B).

Beispiel C

Pyricularia-Test (Reis) /systemisch

Lösungsmittel: 12,5 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

In diesem Test zeigen die erfindungsgemäßen Stoffe (2), (3), (7) und (8) eine bessere Wirksamkeit als die Vergleichssubstanzen (A) und (B).

## Ansprüche

1. Substituierte Hydroxy-azolyl-ketone der Formel

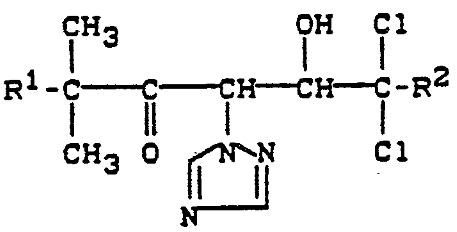 (I)

in welcher
$R^1$ für geradkettiges oder verzweigtes Alkyl mit 2 bis 5 Kohlenstoffatomen oder Alkenyl mit 2 bis 5 Kohlenstoffatomen steht und
$R^2$ für Wasserstoff, Halogen oder gegebenenfalls einfach oder mehrfach durch Halogen substituiertes geradkettiges oder verzweigtes Alkyl mit 1 bis 5 Kohlenstoffatomen steht,
sowie deren Säureadditions-Salze und Metallsalz-Komplexe.

2. Substituierte Hydroxy-azolyl-ketone der Formel (I) gemäß Anspruch 1, in denen
$R^1$ für geradkettiges oder verzweigtes Alkyl mit 2 bis 4 Kohlenstoffatomen oder Alkenyl mit 2 bis 4 Kohlenstoffatomen steht und
$R^2$ für Wasserstoff, Fluor oder Chlor, gegebenenfalls einfach oder mehrfach durch Fluor und/oder Chlor substituiertes geradkettiges oder verzweigtes Alkyl mit 1 bis 4 Kohlenstoffatomen steht.

3. Substituierte Hydroxy-azolyl-ketone der Formel (I) gemäß Anspruch 1, in denen
$R^1$ für geradkettiges oder verzweigtes Alkyl mit 2 oder 3 Kohlenstoffatomen, Vinyl oder Allyl steht und
$R^2$ für Wasserstoff, Chlor, gegebenenfalls einfach oder mehrfach durch Chlor substituiertes geradkettiges oder verzweigtes Alkyl mit 1 bis 3 Kohlenstoffatomen steht.

4. Verfahren zur Herstellung von substituierten Hydroxy-azolyl-ketonen der Formel

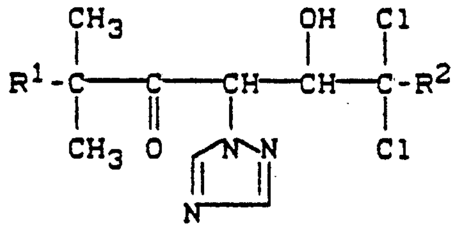
(I)

in welcher
$R^1$ für geradkettiges oder verzweigtes Alkyl mit 2 bis 5 Kohlenstoffatomen oder Alkenyl mit 2 bis 5 Kohlenstoffatomen steht und
$R^2$ für Wasserstoff, Halogen oder gegebenenfalls einfach oder mehrfach durch Halogen substituiertes geradkettiges oder verzweigtes Alkyl mit 1 bis 5 Kohlenstoffatomen steht,
sowie von deren Säureadditions-Salzen und Metallsalz-Komplexen,
dadurch gekennzeichnet, daß man α-Azolylketone der Formel (II)

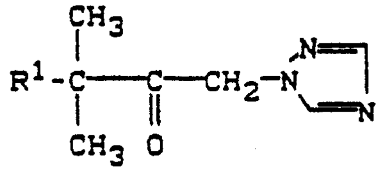
(II)

in welcher
$R^1$ die oben angegebene Bedeutung hat,
mit Aldehyden der Formel (III)

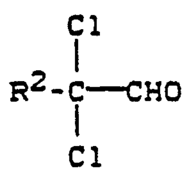
(III)

in welcher
$R^2$ die oben angegebene Bedeutung hat,
oder mit Hydraten oder Halbacetalen von Aldehyden der Formel (III)
in Gegenwart eines Verdünnungsmittels und gegebenenfalls in Gegenwart eines Katalysators umsetzt und gegebenenfalls anschließend an die so erhaltenen Verbindungen der Formel (I) eine Säure oder ein Metallsalz addiert.

5. Fungizide Mittel, gekennzeichnet durch einen Gehalt an mindestens einem substituierten Hydroxy-azolylketon der Formel (I) gemäß Anspruch 1 bzw. an einem Säureadditions-Salz oder Metallsalz-Komplex eines substituierten Hydroxy-azolyl-ketons der Formel (I).

6. Verwendung von substituierten Hydroxy-azolyl-ketonen der Formel (I) gemäß Anspruch 1 bzw. von deren Säureadditions-Salzen oder Metallsalz-Komplexen zur Bekämpfung von Pilzen.

7. Verfahren zur Bekämpfung von Pilzen, dadurch gekennzeichnet, daß man substituierte Hydroxy-azolylketone der Formel (I) gemäß Anspruch 1 bzw. deren Säureadditions-Salze oder Metallsalz-Komplexe auf die Pilze und/oder deren Lebensraum ausbringt.

8. Verfahren zur Herstellung von fungiziden Mitteln, dadurch gekenneichnet, daß man substituierte Hydroxy-azolyl-ketone der Formel (I) gemäß Anspruch 1 bzw. deren Säureadditions-Salze oder Metallsalz-Komplexe mit Streckmitteln und/oder oberflächenaktiven Stoffen vermischt.

Europäisches Patentamt

# EUROPÄISCHER RECHERCHENBERICHT

Nummer der Anmeldung

EP 88 10 0651

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 007 505 (BAYER AG)<br>* Beispiele 1,6,26,37,42,49,52,56; Ansprüche *<br>--- | 1,4-8 | C 07 D 249/08<br>A 01 N 43/653 |
| A | EP-A-0 080 102 (BAYER AG)<br>* Ansprüche 1,3-7 *<br>----- | 1,4-8 | |
| | | | **RECHERCHIERTE SACHGEBIETE (Int. Cl.4)** |
| | | | C 07 D 249/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 17-05-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)